# EUROPÄISCHE PATENTSCHRIFT

(11) **EP 2 069 517 B1**
(45) Veröffentlichungstag und Bekanntmachung des Hinweises auf die Patenterteilung: **23.02.2011**
(21) Anmeldenummer: 07803441.0
(22) Anmeldetag: 12.09.2007
(51) Int. Cl.: C12P 13/02, C12P 41/00

(54) **VERFAHREN ZUR HERSTELLUNG VON OPTISCH AKTIVEM 2-BENZYLOXYCYCLOHEXYLAMIN**
METHOD FOR PRODUCING OPTICALLY ACTIVE 2-BENZYLOXYCYCLOHEXYLAMINE
PROCÉDÉ POUR LA PRODUCTION DE 2-BENZYLOXYCYCLOHEXYLAMINE OPTIQUEMENT ACTIVE

(30) Priorität: 13.09.2006 EP 06120597
(43) Veröffentlichungstag der Anmeldung: 17.06.2009
(73) Patentinhaber: BASF SE, 67056 Ludwigshafen (DE)
(72) Erfinder: DITRICH, Klaus, 67161 Gönnheim (DE); REUTHER, Ute, 67281 Bissersheim (DE); BARTSCH, Michael, 67433 Neustadt (DE)
(86) Internationale Anmeldenummer: PCT/EP2007/059594
(87) Internationale Veröffentlichungsnummer: WO 2008/031852

(56) Entgegenhaltungen:
- WO-A-95/08636
- WO-A-96/23894
- GOTOR V ET AL: "SYNTHESIS OF OPTICALLY ACTIVE AMIDES FROM BETA-FURYL AND BETA-PHENYL ESTERS BY WAY OF ENZYMATIC AMINOLYSIS" JOURNAL OF THE CHEMICAL SOCIETY, PERKIN TRANSACTIONS 1, CHEMICAL SOCIETY. LETCHWORTH, GB, Januar 1993 (1993-01), Seiten 2453-2456, XP002000855 ISSN: 0300-922X
- SCHLICHTER W H ET AL: "ASYMMETRIC REDUCTIVE AMINATION OF CYCLOALKANONES, XIII: ENANTIOSELECTIVE AMIDOAMINATION: A NEW REGIOSPECIFIC STRATEGY FOR THE SYNTHESIS OF CHIRAL CYCLOHEXANE-1,2-DIAMINO-DERIVATIVES" ARCHIV DER PHARMAZIE, VCH VERLAGSGESELLSCHAFT MBH, WEINHEIM, DE, Bd. 326, Nr. 7, 1993, Seiten 429-436, XP000992867 ISSN: 0365-6233 in der Anmeldung erwähnt
- NISHI T ET AL: "STUDIES ON 2-OXOQUINOLINE DERIVATIVES AS BLOOD PLATELET AGGREGATION INHIBITORS. IV. SYNTHESIS AND BIOLOGICAL ACTIVITY OF THE METABOLITES OF 6-[4-(1-CYCLOHEXL-1H-5-TETRAZOLYL)BUTOXY]- 2-OXO-1,2,3,4-TETRAHYDROQUINOLINE (OPC-13013)" CHEMICAL AND PHARMACEUTICAL BULLETIN, TOKYO, JP, Bd. 33, Nr. 3, 1985, Seiten 1140-1147, XP001247898 ISSN: 0009-2363

## Beschreibung

Die vorliegende Erfindung betrifft ein Verfahren zur Herstellung optisch aktiver trans-Stereoisomere von 2-Benzyloxycyclohexylamin.

Optisch aktive Verbindungen besitzen vor allem im Bereich der pharmazeutischen Industrie immense Bedeutung, da häufig nur ein bestimmtes optisch aktives Isomer therapeutisch aktiv ist. Somit besteht ein stetig wachsender Bedarf an optisch aktiven Verbindungen als Edukte für die enantioselektive Synthese von Wirkstoffen. Einer dieser Schlüsselbausteine für die Synthese neuer Wirkstoffe ist das optisch aktive trans-2-Benzyloxycyclohexylamin.

Die WO 96/23894 beschreibt allgemein ein Verfahren zur Racematspaltung primärer und sekundärer heteroatomsubstituierter Amine durch Umsetzung mit einem Ester in Gegenwart einer Hydrolase und anschließender Trennung des enantioselektiv acylierten Enantiomers des heteroatomsubstituierten Amins von dem nicht umgesetzten Enantiomer. Die Enantiomerenreinheiten der Produkte sind jedoch noch nicht zufriedenstellend.

Aufgabe der vorliegenden Erfindung war es, ein effizientes Verfahren zur Herstellung von im Wesentlichen enantiomerenreinen trans- Stereoisomeren von 2-Benzyloxycyclohexylamin bereitzustellen. Insbesondere sollte das Verfahren die Her stellung von im Wesentlichen enantiomerenreinem (R,R)-2-Benzyloxycyclohexyl-amin, v.a. in einer Enantiomerenreinheit von wenigstens 99 % ee, vorzugsweise wenigstens 99,5 % ee und insbesondere wenigstens 99,9 % ee, erlauben.

Überraschenderweise wurde gefunden, dass man optisch aktives 2-Benzyloxycylohexylamin erhält, wenn man ein Enantiomerengemisch, insbesondere das Racemat dieser Amine, in Gegenwart einer Hydrolase enantioselektiv N-acyliert.

Weiterhin wurde gefunden, dass man die jeweiligen trans-Enantiomere in hochreiner Form erhält, wenn man sie durch Zugabe einer Säure (z.B. Carbonsäure, Mineralsäure) in Form ihrer Säureadditionssalze fällt. Gegenstand der Erfindung ist ein Verfahren zur Herstellung von optisch aktiven Verbindungen der Formeln ((R,R)-I) und/oder ((S,S)-I) 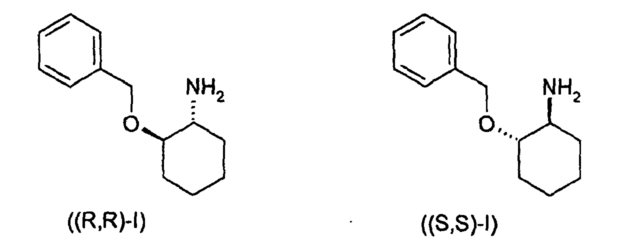 umfassend die folgenden Schritte:
(a) Umsetzung eines Enantiomerengemischs des trans-Stereoisomers der Verbindung der Formel (I) 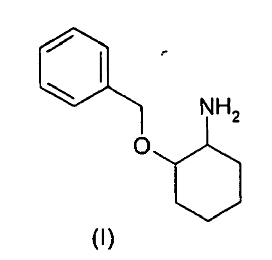 mit einem Acylierungsmittel in Gegenwart einer Hydrolase, wobei man ein Gemisch erhält, in welchem ein Enantiomer des trans-Stereoisomers der Verbindung (I) im Wesentlichen in der acylierten Form vorliegt und das andere Enantiomer des trans-Stereoisomers der Verbindung (I) im Wesentlichen in der nicht acylierten Form vorliegt;
(b) Abtrennung des nicht acylierten Enantiomers des trans-Stereoisomers der Verbindung (I) aus dem in Schritt (a) erhaltenen Gemisch;
(c) Hydrolyse des in Schritt (b) erhaltenen im Wesentlichen acylierten Enantiomers des trans-Stereoisomers der Verbindung (I) zum entsprechenden nicht acylierten Enantiomer des Amins (I);
(d) Überführung der in Schritt (b) oder (c) erhaltenen optisch aktiven Verbindungen der Formeln ((R,R)-I) oder ((S,S)-I) durch Zugabe eines sauren Salzbildners in ihre Ammoniumsalze, wobei der saure Salzbildner in Form einer wässrigen Lösung zugegeben wird und wobei nach Zugabe der wässrigen Lösung das Wasser wenigstens teilweise aus der Reaktionslösung entfernt wird;
(e) Isolierung der Ammoniumsalze; und
(f) Freisetzung der freien Basen der Formeln ((R,R)-I) oder ((S,S)-I) aus den Ammoniumsalzen.

Die im Folgenden beschriebenen bevorzugten Merkmale und Ausführungsformen des efindungsgemäßen Verfahrens gelten jeweils eigenständig oder insbesondere in Kombination miteinander.

Die Deskriptoren "trans" und "cis" beschreiben im Rahmen der vorliegenden Erfindung die relative Anordnung der beiden Substituenten am Cyclohexylring zueinander. Der Ausdruck trans-Stereoisomer umfasst somit (R,R)-2-Benzyloxycyclohexylamin und (S,S)-2-Benzyloxycyclohexylamin. Der Ausdruck cis-Stereoisomer umfasst das andere Diastereomer, bestehend aus (R,S)-2-Benzyloxycyclohexylamin und (S,R)-2-Benzyloxycyclohexylamin.

Der Ausdruck "enantioselektiv" beschreibt eine Umsetzung, bei der die zueinander spiegelbildlichen möglichen Reaktionsprodukte zu ungleichen Teilen gebildet werden. Im Rahmen der vorliegenden Erfindung beschreibt dieser Ausdruck Umsetzungen, bei denen das gewünschte Enantiomer bevorzugt mit wenigstens 95 % ee, besonders bevorzugt mit wenigstens 96 % ee, stärker bevorzugt mit wenigstens 98 % ee, noch stärker bevorzugt mit wenigstens 99 % ee, insbesondere mit wenigstens 99,5 % ee und speziell mit wenigstens 99,9 % ee gebildet wird.

Der Ausdruck "im Wesentlichen enantiomerenrein" beschreibt somit im Rahmen der vorliegenden Erfindung eine Enantiomerenreinheit von jeweils wenigstens 95 % ee, vorzugsweise wenigstens 96 % ee, stärker bevorzugt wenigstens 98 % ee, noch stärker bevorzugt wenigstens 99 % ee, insbesondere wenigstens 99,5 % ee und speziell wenigstens 99,9 % ee.

Die Einheit "% ee" bezieht sich auf den Enantiomerenüberschuss und ist somit ein Maß für die Enantiomerenreinheit, die auch als optische Reinheit bezeichnet wird. Diese errechnet sich aus der Differenz der molaren Anteile der beiden Enantiomere in einer Enantiomerenmischung. Beispielsweise bedeutet 95 % ee, dass der Anteil des Hauptenantiomers in der Enantiomerenmischung 97,5 % beträgt und der Anteil der dazu spiegelbildlichen Verbindung 2,5 % beträgt.

Vorzugsweise wird bei der enantioselektiven Acylierung des trans-Stereoisomers der Verbindung (I) ein Gemisch erhalten, in dem das (S,S)-Enantiomer im Wesentlichen in nicht acylierter Form vorliegt und das (R,R)-Enantiomer im Wesentlichen in acylierter Form enthalten ist.

Der Ausdruck "im Wesentlichen nicht acyliertes Enantiomer" beschreibt, dass wenigstens 95%, vorzugsweise wenigstens 97%, insbesondere wenigstens 98% dieses Enantiomers nicht acyliert sind. Sinngemäß gilt für den Ausdruck im Wesentlichen acyliertes Enantiomer", dass wenigstens 95%, vorzugsweise wenigstens 97%, besonders bevorzugt wenigstens 98%, dieses Enantiomers acyliert vorliegen.

Der Ausdruck "im Wesentlichen nicht acyliertes (S,S)-Enantiomer der Verbindung (I)" soll dementsprechend so verstanden werden, dass wenigstens 95%, vorzugsweise wenigstens 97%, insbesondere wenigstens 98% des (S,S)-Enantiomers der Verbindung (I) nicht acyliert sind. Sinngemäß gilt für den Ausdruck "im Wesentlichen acyliertes (R,R)-Enantiomer der Verbindung (I)", dass wenigstens 95%, vorzugsweise wenigstens 97%, besonders bevorzugt wenigstens 98%, des (R,R)-Enantiomers der Verbindung (I) acyliert vorliegen.

Bei der in den erfindungsgemäßen Verfahren eingesetzten Hydrolase handelt es sich vorzugsweise um eine Protease und insbesondere um eine Lipase. Diese bewirkt eine selektive N-Acylierung (Amidierung) nur eines der jeweils zwei Enantiomere des trans-Stereoisomers . Besonders bevorzugt bewirkt es die selektive Amidierung des (R,R)-Enantiomers der Verbindung (I).

Die Hydrolase wird vorzugsweise aus einem Mikroorganismus, besonders bevorzugt aus einem Bakterium oder einer Hefe gewonnen. Ebenfalls geeignet sind Hydrolasen, die durch rekombinante Verfahren erhältlich sind. Die Hydrolase kann in gereinigter oder teilweise gereinigter Form oder in Form des Mikroorganismus selbst verwendet werden. Verfahren zur Gewinnung und Aufreinigung von Hydrolasen aus Mikroorganismen sind dem Fachmann hinreichend bekannt, z.B. aus EP-A-1149849 oder EP-A-1069183. Vorzugsweise wird die Hydrolase in gereinigter Form eingesetzt.

Die Hydrolase kann frei (d.h. in nativer Form) oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069183 und der DE-OS 10019377 sowie aus den darin zitierten Literaturstellen bekannt. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaus tauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie).

Bevorzugt werden Lipasen (Triacylglycerolacylhydrolasen; EC 3.1.1.3) eingesetzt. Hierunter bevorzugt sind Lipasen, die aus Bakterien der Gattungen Burkholderia oder Pseudomonas oder aus Hefen der Gattung Candida gewonnen werden.

Beispiele für Burkholderia-Arten sind Burkholderia ambifaria (z. B. Stämme ATCC BAA-244, CCUG 44356, LMG 19182); Burkholderia andropogonis (z. B. Stämme ATCC 23061, CCUG 32772, CFBP 2421, CIP 105771, DSM 9511, ICMP 2807, JCM 10487, LMG 2129, NCPPB 934, NRRL B-14296); Burkholderia caledonica (z. B. Stämme W50D, CCUG 42236, CIP 107098, LMG 19076); Burkholderia caribensis (z. B. Stämme MWAP 64, CCUG 42847, CIP 106784, DSM 13236, LMG 18531); Burkholderia caryophylli (z.B. Stämme ATCC 25418, CCUG 20834, CFBP 2429, CFBP 3818, CIP 105770, DSM 50341, HAMBI 2159, ICMP 512, JCM 9310, JCM 10488, LMG 2155, NCPPB 2151); Burkholderia cepacia (z. B. Stämme Ballard 717, 717-ICPB 25, ATCC 25416, CCUG 12691, CCUG 13226, CFBP 2227, CIP 80.24, DSM 7288, HAMBI 1976, ICMP 5796, IFO 14074, JCM 5964, LMG 1222, NCCB 76047, NCPPB 2993, NCTC 10743, NRRL B-14810); Burkholderia cocovenenans (z. B. Stämme ATCC 33664, CFBP 4790, DSM 11318, JCM 10561, LMG 11626, NCIMB 9450); Burkholderia fungorum (z. B. Stämme Croize P763-2, CCUG 31961, CIP 107096, LMG 16225); Burkholderia gladioli (z. B. Stämme ATCC 10248, CCUG 1782, CFBP 2427, CIP 105410, DSM 4285, HAMBI 2157, ICMP 3950, IFO 13700, JCM 9311, LMG 2216, NCCB 38018, NCPPB 1891, NCTC 12378, NRRL B-793); Burkholderia glathei (z. B. Stämme ATCC 29195, CFBP 4791, CIP 105421, DSM 50014, JCM 10563, LMG 14190); Burkholderia glumae (z.B. Stämme ATCC 33617, CCUG 20835, CFBP 4900, CFBP 2430, CIP 106418, DSM 9512, ICMP 3655, LMG 2196, NCPPB 2981, NIAES 1169); Burkholderia graminis (z. B. Stämme C4D1 M, ATCC 700544, CCUG 42231, CIP 106649, LMG 18924); Burkholderia kururiensis (z. B. Stämme KP 23, ATCC 700977, CIP 106643, DSM 13646, JCM 10599, LMG 19447); Burkholderia mallei (z. B. Stämme ATCC 23344, NCTC 12938); Burkholderia multivorans (z. B. Stämme ATCC BAA-247, CCUG 34080, CIP 105495, DSM 13243, LMG 13010, NCTC 13007); Burkholderia norimbergensis (z. B. Stämme R2, ATCC BAA-65, CCUG 39188, CFBP 4792, DSM 11628, CIP 105463, JCM 10565, LMG 18379); Burkholderia phenazinium (z. B. Stämme ATCC 33666, CCUG 20836, CFBP 4793, CIP 106502, DSM 10684, JCM 10564, LMG 2247, NCIB 11027); Burkholderia pikettii (z. B. Stämme ATCC 27511, CCUG 3318, CFBP 2459, CIP 73.23, DSM 6297, HAMBI 2158, JCM 5969, LMG 5942, NCTC 11149); Burkholderia plantarü (z. B. Stämme AZ 8201, ATCC 43733, CCUG 23368, CFBP 3573, CFBP 3997, CIP 105769, DSM 9509, ICMP 9424 JCM 5492, LMG 9035, NCPPB 3590, NIAES 1723); Burkholderia pseudomallei (z. B. Stämme WRAIR 286, ATCC 23343, NCTC 12939); Burkholderia pyrrocinia (z. B. Stämme ATCC 15958, CFBP 4794, CIP 105874, DSM 10685, LMG 14191); Burkholderia sacchari (z. B. Stämme CCT 6771, CIP 107211, IPT 101, LMG 19450); Burkholderia solanacearum (z. B. Stämme A. Kelman 60-1, ATCC 11696, CCUG 14272, CFBP 2047, CIP 104762, DSM 9544, ICMP 5712, JCM 10489, LMG 2299, NCAIM B.01459, NCPPB 325, NRRL B-3212); Burkholderia stabilis (z. B. Stämme ATCC BAA-67, CCUG 34168, CIP 106845, LMG 14294, NCTC 13011); Burkholderia thailandensis (z. B. Stämme E 264, ATCC 700388, CIP 106301, DSM 13276); Burkholderia ubonensis (z. B. Stämme EY 3383, CIP 107078, NCTC 13147); Burkholderia vandii (z. B. Stämme VA-1316, ATCC 51545, CFBP 4795, DSM 9510, JCM 7957, LMG 16020); Burkholderia vietnamiensis (z. B. Stämme TVV 75, ATCC BAA-248, CCUG 34169, CFBP 4796, CIP 105875, DSM 11319, JCM 10562, LMG 10929).

Beispiele für Pseudomonas-Arten sind Pseudomonas aeruginosa (z. B. Stämme ATCC 10145, DSM 50071), Pseudomonas agarici (z. B. Stämme ATCC 25941, DSM 11810), Pseudomonas alcaligenes (z. B. Stämme ATCC 14909, DSM 50342), Pseudomonas amygdali (z. B. Stämme ATCC 337614, DSM 7298), Pseudomonas anguiliseptica (z. B. Stämme ATCC 33660, DSM 12111), Pseudomonas antimicrobica (z. B. Stämme DSM 8361, NCIB 9898, LMG 18920), Pseudomonas aspleni (z. B. Stämme ATCC 23835, CCUG 32773), Pseudomonas aurantiaca (z. B. Stämme ATCC 33663, CIP 106710), Pseudomonas aureofaciens (z. B. Stämme ATCC 13985, CFBP 2133), Pseudomonas avellanae (z. B. Stämme DSM 11809, NCPPB 3487), Pseudomonas azotoformans (z. B. Stämme CIP 106744, JCM 7733), Pseudomonas balearica (z. B. Stämme DSM 6083, CIP 105297)), Pseudomonas beijerinsckii (z. B. Stämme ATCC 19372, DSM 6083), Pseudomonas beteli (z. B. Stämme ATCC 19861, CFBP 4337), Pseudomonas boreopolis (z. B. Stämme ATCC 33662, CIP 106717), Pseudomonas carboxyhydrogena (z. B. Stämme ATCC 29978, DSM 1083), Pseudomonas caricapapayae (z. B. Stämme ATCC 33615, CCUG 32775), Pseudomonas cichorii (z. B. Stämme ATCC 10857, DSM 50259), Pseudomonas cissicola (z. B. Stämme ATCC 33616, CCUG 18839), Pseudomonas citronellolis (z. B. Stämme ATCC 13674, DSM 50332), Pseudomonas coronafaciens (z. B. Stämme DSM 50261, DSM 50262), Pseudomonas corrugata (z. B. Stämme ATCC 29736, DSM 7228), Pseudomonas doudoroffii (z. B. Stämme ATCC 27123, DSM 7028), Pseudomonas echinoides (z. B. Stämme ATCC 14820, DSM 1805), Pseudomonas elongata (z. B. Stämme ATCC 10144, DSM 6810), Pseudomonas ficuserectae (z. B. Stämme ATCC 35104, CCUG 32779), Pseudomonas flavescens (z. B. Stämme ATCC 51555, DSM 12071), Pseudomonas flectens (z. B. Stämme ATCC 12775, CFBB 3281), Pseudomonas fluorescens (z. B. Stämme ATCC 13525, DSM 50090), Pseudomonas fragi (z. B. Stämme ATCC 4973, DSM 3456), Pseudomonas fulva (z. B. Stämme ATCC 31418, CIP 106765), Pseudomonas fuscovaginae (z. B. Stämme CCUG 32780, DSM 7231), Pseudomonas gelidicola (z. B. Stämme CIP 106748), Pseudomonas geniculata (z. B. Stämme ATCC 19374, LMG 2195), Pseudomonas glathei (z. B. Stämme ATCC 29195, DSM 50014), Pseudomonas halophila (z. B. Stämme ATCC 49241, DSM 3050), Pseudomonas hibiscicola (z. B. Stämme ATCC 19867, LMG 980), Pseudomonas huttiensis (z. B. Stämme ATCC 14670, DSM 10281), Pseudomonas iners (z. B. Stamm CIP 106746), Pseudomonas lancelota (z. B. Stämme ATCC 14669, CFBP 5587), Pseudomonas lemoignei (z. B. Stämme ATCC 17989, DSM 7445), Pseudomonas lundensis (z. B. Stämme ATCC 19968, DSM 6252), Pseudomonas luteola (z. B. Stämme ATCC 43273, DSM 6975), Pseudomonas marginalis (z. B. Stämme ATCC 10844, DSM 13124), Pseudomonas meliae (z. B. Stämme ATCC 33050, DSM 6759), Pseudomonas mendocina (z. B. Stämme ATCC 25411, DSM 50017), Pseudomonas mucidolens (z. B. Stämme ATCC 4685, CCUG 1424), Pseudomonas monteilli (z. B. Stämme ATCC 700476, DSM 14164), Pseudomonas nautica (z. B. Stämme ATCC 27132, DSM 50418), Pseudomonas nitroreducens (z. B. Stämme ATCC 33634, DSM 14399), Pseudomonas oleovorans (z. B. Stämme ATCC 8062, DSM 1045), Pseudomonas oryzihabitans (z. B. Stämme ATCC 43272, DSM 6835), Pseudomonas pertucinogena (z. B. Stämme ATCC 190, CCUG 7832), Pseudomonas phenazinium (z. B. Stämme ATCC 33666, DSM 10684), Pseudomonas pictorum (z. B. Stämme ATCC 23328, LMG 981), Pseudomonas pseudoalcaligenes (z. B. Stämme ATCC 17440, DSM 50188), Pseudomonas putida (z. B. Stämme ATCC 12633, DSM 291), Pseudomonas pyrrocinia (z. B. Stämme ATCC 15958, DSM 10685), Pseudomonas resinovorans (z. B. Stämme ATCC 14235, CCUG 2473), Pseudomonas rhodesiae (z. B. Stämme CCUG 38732, DSM 14020), Pseudomonas saccharophila (z. B. Stämme ATCC 15946, DSM 654), Pseudomonas savastanoi (z. B. Stämme ATCC 13522, CFBP 1670), Pseudomonas spinosa (z. B. Stämme ATCC 14606), Pseudomonas stanieri (z. B. Stämme ATCC 27130, DSM 7027), Pseudomonas straminae (z. B. Stämme ATCC 33636, CIP 106745), Pseudomonas stutzeri (z. B. Stämme ATCC 17588, DSM 5190), Pseudomonas synxantha (z. B. Stämme ATCC 9890, CFBP 5591), Pseudomonas syringae (z. B. Stämme ATCC 19310, DSM 6693), Pseudomonas syzygii (z. B. Stämme ATCC 49543, DSM 7385), Pseudomonas taetrolens (z. B. Stämme ATCC 4683, CFBP 5592), Pseudomonas tolaasii (z. B. Stämme ATCC 33618, CCUG 32782), Pseudomonas veronii (z. B. Stämme ATCC 700272, DSM 11331), Pseudomonas viridiflava (z. B. Stämme ATCC 13223, DSM 11124), Pseudomonas vulgaris, Pseudomonas wisconsinensis und Pseudomonas spec. DSM 8246. Davon sind Lipasen aus Burkholderia glumae, Burkholderia plantarii, Burkholderia cepacia, Pseudomonas aeruginosa, Pseudomonas fluorescens, Pseudomonas fragi, Pseudomonas luteola, Pseudomonas vulgaris, Pseudomonas wisconsinensis und Pseudomonas spec. DSM 8246 bevorzugt. Besonders bevorzugt sind Lipasen aus Pseudomonas spec. DSM 8246.

Beispiele für Candida-Arten sind Candida albomarginata (z.B. Stamm DSM 70015), Candida antarctica (z.B. Stamm DSM 70725), Candida bacarum (z.B. Stamm DSM 70854), Candida bogoriensis (z.B. Stamm DSM 70872), Candida boidinii (z.B. Stämme DSM 70026, 70024, 70033, 70034), Candida bovina (z.B. Stamm DSM 70156), Candida brumptii (z.B. Stamm DSM 70040), Candida cacaoi (z.B. Stamm DSM 2226), Candida cariosilignicola (z.B. Stamm DSM 2148), Candida chalmersii (z.B. Stamm DSM 70126), Candida ciferii (z.B. Stamm DSM 70749), Candida cylindracea (z.B. Stamm DSM 2031), Candida ernobii (z.B. Stamm DSM 70858), Candida famata (z.B. Stamm DSM 70590), Candida freyschussii (z.B. Stamm DSM 70047), Candida friederichii (z.B. Stamm DSM 70050), Candida glabrata (z.B. Stämme DSM 6425, 11226, 70614, 70615), Candida guillermondi (z.B. Stämme DSM 11947, 70051, 70052), Candida haemulonii (z.B. Stamm DSM 70624), Candida inconspicua (z.B. Stamm DSM 70631), Candida ingens (z.B. Stämme DSM 70068, 70069), Candida intermedia (z.B. Stamm DSM 70753), Candida kefyr (z.B. Stämme DSM 70073, 70106), Candida krusei (z.B. Stämme DSM 6128, 11956, 70075, 70079, 70086), Candida lactiscondensi (z.B. Stamm DSM 70635), Candida lambica (z.B. Stämme DSM 70090, 70095), Candida lipolytica (z.B. Stämme DSM 1345, 3286, 8218, 70561 oder 70562), Candida lusitaniae (z.B. Stamm DSM 70102), Candida macedoniensis (z.B. Stamm DSM 70106), Candida magnoliae (z.B. Stämme DSM 70638, 70639), Candida membranaefaciens (z.B. Stamm DSM 70109), Candida multigemnis (z.B. Stamm DSM 70862), Candida mycoderma (z.B. Stamm DSM 70184), Candida nemodendra (z.B. Stamm DSM 70647), Candida nitratophila (z.B. Stamm DSM 70649), Candida norvegica (z.B. Stamm DSM 70862), Candida parapsilosis (z.B. Stämme DSM 5784,4237, 11224, 70125, 70126), Candida pelliculosa (z.B. Stamm DSM 70130), Candida pini (z.B. Stamm DSM 70653), Candida pulcherrima (z.B. Stamm DSM 70336), Candida punicea (z.B. Stamm DSM 4657), Candida pustula (z.B. Stamm DSM 70865), Candida rugosa (z.B. Stamm DSM 70761), Candida sake (z.B. Stamm DSM 70763), Candida silvicola (z.B. Stamm DSM 70764), Candida solani (z.B. Stamm DSM 3315), Candida sp. (z.B. Stamm DSM 1247), Candida spandovensis (z.B. Stamm DSM 70866), Candida succiphila (z.B. Stamm DSM 2149), Candida utilis (z.B. Stämme DSM 2361, 70163 oder 70167), Candida valida (z.B. Stämme DSM 70169, 70178, 70179), Candida versatilis (z.B. Stamm DSM 6956), Candida vini (z.B. Stamm DSM 70184) und Candida zeylanoides (z.B. Stamm DSM 70185).

Besonders bevorzugt werden in dem erfindungsgemäßen Verfahren Lipasen aus Hefen der Gattung Candida eingesetzt, insbesondere aus Candida antarctica. Demzufolge wird in einer speziellen Ausführungsform deserfindungsgemäßen verfarens Lipase B aus Candida antarctica verwendet. Bevorzugt ist die immobilisierte Form dieser Lipase, z.B. die auf Acrylharz immobilisierte Lipase B aus Candida antarctica, die z.B. unter der Bezeichnung "Novozyme 435®" kommerziell erhältlich ist.

Die in dem erfindungsgemäßen Verfahren bei der enantioselektiven Acylierung einzusetzende Menge an Hydrolase hängt von deren Art und der Aktivität der Enzympräparation ab. Die für die Reaktion optimale Enzymmenge kann leicht durch einfache Vorversuche ermittelt werden. In der Regel werden etwa 1000 units Hydrolase pro mmol des zu trennenden Enantiomerengemischs eingesetzt.

Die Einheit "units" beschreibt die Aktivität der Hydrolase und bezieht sich auf die von der Hydrolase unter definierten Standardbedingungen umgesetzte Menge einer Referenzverbindung.

Die in dem erfindungsgemäßen Verfahren eingesetzten Acylierungsmittel sind vorzugsweise unter solchen ausgewählt, in denen die Säurekomponente ein elektronenreiches Heteroatom, das beispielsweise ausgewählt ist unter Fluor-, Stickstoff-, Sauerstoff- und Schwefelatomen, in Nachbarschaft zum Carbonylkohlenstoffatom trägt. Vorzugsweise handelt es sich bei dem Acylierungsmittel um einen Ester. Besonders bevorzugt ist das Acylierungsmittel ausgewählt unter Estern, deren Säurekomponente in α-, β- oder γ-Stellung zum Carbonyl-Kohlenstoffatom eine sauerstoff-, stickstoff-, fluor- oder schwefelhaltige Gruppe trägt. Besonders bevorzugt sind solche Acylierungsmittel, in denen das Heteroatom selbst in α-, β- oder γ-Stellung und insbesondere in α-Stellung zum Carbonylkohlenstoffatom gebunden ist.

Bei der sauerstoffhaltigen Gruppe handelt es sich beispielsweise um eine Hydroxygruppe oder um eine Alkoxygruppe. Bei der stickstoffhaltigen Gruppe handelt es sich beispielsweise um Aminogruppen, während es sich bei der schwefelhaltigen Gruppe um die Thiolgruppe (SH) oder um Alkylthiogruppen handeln kann.

Die Alkoholkomponente des Esters leitet sich vorzugsweise von linearen oder verzweigten C₁-C₁₀-Alkoholen, die substituiert oder vorzugsweise unsubstituiert sein können, ab. Besonders bevorzugt leitet sich die Alkoholkomponente jedoch von sekundären Alkoholen ab, wie Isopropanol, 2-Butanol, 2- oder 3-Pentanol und dergleichen. Speziell leitet sie sich von Isopropanol ab.

Besonders geeignete Ester sind solche der Formel (III) 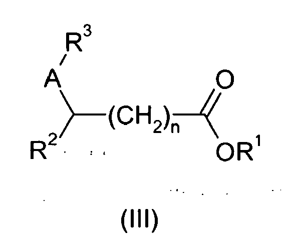 worin
- R¹: für C₁-C₁₀-Alkyl steht,
- R²: für Wasserstoff oder C₁-C₁₀-Alkyl steht,
- R³: für Wasserstoff, C₁-C₁₀-Alkyl oder Phenyl, das gegebenenfalls durch NH₂, OH, C₁-C₄-Alkoxy oder Halogen substituiert ist, steht,
- A: für O, S oder NR⁴, bevorzugt für O steht,
- R⁴: für Wasserstoff, C₁-C₁₀-Alkyl oder Phenyl, das gegebenenfalls durch NH₂, OH, C₁-C₄-Alkoxy oder Halogen substituiert ist, steht, und
- n: für 0, 1 oder 2 steht.

Bevorzugt steht R¹ für lineares oder verzweigtes C₁-C₄-Alkyl. Insbesondere leitet sich R¹ von sekundären Alkoholen ab und steht dementsprechend besonders bevorzugt für eine über ein tertiäres Kohlenstoffatom gebundene C₃-C₄-Alkylgruppe, wie Isopropyl oder 2-Butyl. Speziell steht R¹ für Isopropyl.

Bevorzugt steht R² für Wasserstoff oder C₁-C₄-Alkyl und insbesondere für Wasserstoff.

Bevorzugt steht R³ für C₁-C₄-Alkyl, besonders bevorzugt für Methyl oder Ethyl und speziell für Methyl.

Bevorzugt steht A für O.

Der Ausdruck "C₁-C₄-Alkyl" beschreibt im Rahmen der vorliegenden Erfindung einen linearen oder verzweigten Alkylrest mit 1 bis 4 Kohlenstoffatomen, wie Methyl, Ethyl, Propyl, Isopropyl, n-Butyl, sec-Butyl, Isobutyl oder tert-Butyl.

Der Ausdruck "C₁-C₁₀-Alkyl" steht für einen linearen oder verzweigten Alkylrest mit 1 bis 10 Kohlenstoffatomen. Beispiele hierfür sind, neben den zuvor genannten C₁-C₄-Alkylresten, Pentyl, Neopentyl, Hexyl, Heptyl, Octyl, 2-Ethylhexyl, Nonyl, Neononyl, Decyl und Neodecyl.

Der Ausdruck "C₁-C₄-Alkoxy" beschreibt einen über Sauerstoff gebundenen Alkylrest mit 1 bis 4 Kohlenstoffatomen. Beispiele hierfür sind Methoxy, Ethoxy, Propoxy, Isopropoxy, n-Butoxy, sec-Butoxy, Isobutoxy und tert-Butoxy.

Der Ausdruck "C₁-C₁₀-Alkohol" steht für einen aliphatischen Kohlenwasserstoff mit 1 bis 10 Kohlenstoffatomen, der durch wenigstens eine Hydroxygruppe substituiert ist. Vorzugsweise steht C₁-C₁₀-Alkohol für ein mit einem Hydroxyrest substituiertes Alkan. Beispiele hierfür sind Methanol, Ethanol, Propanol, Isopropanol, Butanol, sec-Butanol, Isobutanol, tert-Butanol, Pentanol, Hexanol, Heptanol, Octanol, 2-Ethylhexanol, Nonanol und Decanol.

Halogen steht im Rahmen der vorliegenden Erfindung vorzugsweise für Fluor, Chlor oder Brom, besonders bevorzugt für Fluor oder Chlor.

Vorzugsweise setzt man bei der enantioselektiven Acylierung in den erfindungsgemäßen Verfahren 1 bis 3 Moläquivalente, besonders bevorzugt 1 bis 2 Moläquivalente, stärker bevorzugt 1 bis 1,5 Moläquivalente und insbesondere 1 bis 1,2 Moläquivalente des Acylierungsmittels, bezogen auf die Menge an Enantiomer der Verbindung der Formel (I), das acyliert wird, ein. Der Ausdruck "Moläquivalente" bezieht sich hier auf die Anzahl der Carboxylgruppen des Acylierungsmittels in Mol, die mit einem Mol des Enantiomers der Verbindung (I), das acyliert wird, reagieren können. Dementsprechend setzt man bei Verwendung eines Esters der Formel (III) vorzugsweise 1 bis 3 mol, besonders bevorzugt 1 bis 2 mol, stärker bevorzugt 1 bis 1,5 mol und insbesondere 1 bis 1,2 mol Ester pro mol des Enantiomers der Verbindung der Formel (I), das acyliert wird, ein. Alternativ setzt man bei der Verwendung der Ester der Formel (III) vorzugsweise 0,5 bis 1,5 mol, besonders bevorzugt 0,5 bis 1 mol, stärker bevorzugt 0,5 bis 0,75 und insbesondere 0,5 bis 0,6 mol Ester, bezogen auf 1 mol des zu trennenden Enantiomerengemischs, ein, insbesondere wenn es sich bei diesem Gemisch um das Racemat handelt.

In einer bevorzugten Ausführungsform des efindungsgemäßen Verfahrens erfolgt die Umsetzung in Schritt (a) des Verfahrens in Substanz, d.h. ohne Zusatz von wässrigem oder organischem Lösungsmittel.

In einer alternativ bevorzugten Ausführungsform wird die Acylierung in dem erfindungsgemäßen Verfahren in einem nicht-wässrigen Reaktionsmedium durchgeführt. Unter nicht-wässrigen Reaktionsmedien sollen Reaktionsmedien verstanden werden, die weniger als 1 Gew.-%, vorzugsweise weniger als 0,5 Gew.-% Wasser, besonders bevorzugt weniger als 0,1 Gew.-% Wasser und insbesondere weniger als 0,05 Gew.-% Wasser, bezogen auf das Gesamtgewicht des Reaktionsmediums, enthalten. Vorzugsweise wird bei dieser Ausführungsform die Acylierung in einem organischen Lösungsmittel durchgeführt. Geeignete Lösungsmittel sind beispielsweise aliphatische und alicyclische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit 1 oder 2 Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Dioxan, oder Gemische der vorgenannten Lösungsmittel. Besonders bevorzugt werden die vorgenannten Ether und aromatischen Kohlenwasserstoffe eingesetzt. Insbesondere verwendet man Toluol.

Die Umsetzung der Verbindungen der Formel (I) mit dem Acylierungsmittel erfolgt in der Regel bei einer Reaktionstemperatur unterhalb der Deaktivierungstemperatur der eingesetzten Hydrolase und vorzugsweise bei wenigstens -10°C. Besonders bevorzugt liegt sie im Bereich von 0 bis 80°C, insbesondere von 10 bis 40°C. Speziell erfolgt die Umsetzung bei Raumtemperatur.

Zur Durchführung kann man beispielsweise ein Enantiomerengemisch der trans-Stereoisomere der Verbindungen der Formel (I) mit der Hydrolase, dem Acylierungsmittel und gegebenenfalls dem Lösungsmittel vorlegen und das Gemisch durchmischen, z.B. durch Rühren oder Schütteln. Es ist aber auch möglich, die Hydrolase in einem Reaktor, beispielsweise in einer Säule, zu immobilisieren, und durch den Reaktor eine das Enantiomerengemisch und das Acylierungsmittel enthaltende Mischung zu leiten. Hierzu kann man die Mischung im Kreislauf durch den Reaktor leiten, bis der gewünschte Umsatz erreicht ist. Dabei werden die Carboxylgruppen des Acylierungsmittels sequenziell in Amide desjenigen Enantiomers der Verbindung (I) überführt, das enantioselektiv acyliert wird, während das andere Enantiomer im Wesentlichen unverändert bleibt. In der Regel wird man die Acylierung bis zu einem Umsatz von wenigstens 95%, bevorzugt von wenigstens 98% und insbesondere von wenigstens 99 %, bezogen auf das in der Mischung enthaltene Enantiomer der Verbindung (I), welches enantioselektiv acyliert wird, führen. Das Fortschreiten der Reaktion, d.h. die sequenzielle Amidbildung, kann dabei durch übliche Methoden wie Gaschromatographie oder HPLC (High Performance Liquid Chromatography) verfolgt werden.

Die Aufarbeitung des Reaktionsgemischs kann in üblicher Weise erfolgen, beispielsweise, indem man gegebenenfalls die Hydrolase aus dem Reaktionsgemisch abtrennt, z.B. durch Abfiltrieren oder Abzentrifugieren, aus dem Filtrat bzw. Zentrifugat das Lösungsmittel entfernt und den Rückstand anschließend einer Trennoperation unterwirft.

In einer speziellen Ausführungsform des erfindungsgemäßen Verfahrens handelt es sich bei dem eingesetzten Enantiomerengemisch um das Racemat des Amins (I); Gemische, in denen eines der Enantiomere angereichert ist, sind jedoch ebenso geeignet.

Durch die enantioselektive Umsetzung des Enantiomerengemisches der trans- Isomere der Verbindung (I) entsteht ein Reaktionsprodukt, das ein im Wesentlichen acyliertes Enantiomer (d.h. Amid) der Verbindung (I) und das im Wesentlichen nicht acylierte entgegengesetzte Enantiomer enthält. Dieses nun vorliegende Gemisch aus Amin und Amid lässt sich mit üblichen Methoden leicht trennen. Geeignete Trennoperationen sind beispielsweise Extraktion, Destillation, Kristallisation oder Chromatographie. Vorzugsweise erfolgt die Trennung des Amins und des Amids destillativ. In einem alternativ bevorzugten Trennverfahren wird das in einem organischen Lösungsmittel gelöste oder suspendierte Reaktionsgemisch mit einem sauren Salzbildner versetzt, wobei sich das Ammoniumsalz des nicht acylierten Enantiomers bildet und präzipitiert. Dieses kann dann durch Filtration oder Zentrifugieren vom Überstand, welcher das Amid des entgegengesetzten Enantiomers der Verbindung (I) enthält, abgetrennt werden. Geeignete saure Salzbildner sind beispielsweise Protonensäuren, insbesondere Mineralsäuren, wie Salzsäure, Bromwasserstoffsäure, Schwefelsäure oder Salpetersäure, aber auch organische Säuren, wie Tritluoressigsäure, Trifluormethansuffonsäure oder para-Toluolsulfonsäure sowie deren Alkalimetallsalze, wobei jedoch Säuren, vor allem Mineralsäuren und insbesondere Salzsäure oder Schwefelsäure, bevorzugt sind. Vorzugsweise wird die Säure in etwa äquimolarer Menge in Bezug auf die Menge an nicht acyliertem Enantiomer eingesetzt, z.B. in einer Menge von 0,9 bis 1,5 mol, vorzugsweise 1 bis 1,2 mol und insbesondere etwa 1 mol, bezogen auf 1 mol des nicht acylierten Enantiomers. Bei mehrprotonigen Säuren, wie Schwefelsäure, beziehen sich die Molvefiältnisse selbstverständlich auf die Anzahl der in der Säure enthaltenen aciden Protonen.

Das abgetrennte, im Wesentlichen nicht acylierte Enantiomer der Verbindung (I) kann gegebenenfalls unter Verwendung von dem Fachmann bekannten Methoden einer weiteren Aufreinigung unterzogen werden. Insbesondere handelt es sich bei dem im Anschluss an die Acylierung abgetrennten nicht acylierten Enantiomer um (S,S)-2-Benzyloxycyclohexylamin ((S,S)-I) [d.h. in Schritt (b) des Verfahrens erhält man insbesondere (S,S)-2-Benzyloxycyclohexylamin ((S,S)-I)].

Liegt das erhaltene im Wesentlichen nicht acylierte Enantiomer der Verbindung (I) aufgrund der Trennmethode in Form des Ammoniumsalzes vor, so wird das Amin aus dem Ammoniumsalz mittels einer geeigneten Base freigesetzt. Geeignete Basen sind beispielsweise Alkalihydroxide, wie Natriumhydroxid oder Kaliumhydroxid, Erdalkalihydroxide, wie Calciumhydroxid oder Magnesiumhydroxid, Alkalicarbonate oder Erdalkalicarbonate, wie Natriumcarbonat, Kaliumcarbonat oder Calciumcarbonat. Bevorzugt verwendet man Alkalihydroxide, wie Natriumhydroxid oder Kaliumhydroxid. Vorzugsweise erfolgt die Neutralisation in einem wässrigen Medium. Zur leichteren Isolierung des Amins wird die Base vorzugsweise in einer solchen Menge eingesetzt, dass die freigesetzte Verbindung in neutraler Form vorliegt. Das gewonnene freie Amin kann anschließend gewünschtenfalls weiteren Reinigungsschritten unterworfen werden.

Das andere Enantiomer, das in dem erfindungsgemäßen Verfahren enantioselektiv acyliert wurde, kann dadurch gewonnen werden, dass man das bei der Acylierung gewonnene im Wesentlichen acylierte Enantiomer der Verbindung (I), d.h. das Amid der Verbindung (I), unter Abspaltung der Acylfunktion hydrolysiert, wobei das entsprechende Enantiomer der Verbindung (I) erhalten wird. Vorzugsweise handelt es sich bei dem erhaltenen Produkt um (R,R)-2-Benzyloxycyclohexylamin ((R,R)-I) [d.h. in Schritt (c) des Vertahrens erhält man vorzugsweise (R,R)-2-Benzyloxycyclohexylamin ((R,R)-I)].

Die Hydrolyse erfolgt dabei in der Regel unter Reaktionsbedingungen, wie sie für die Hydrolyse von Amiden bekannt sind. Reaktionsbedingungen sind beispielsweise in DE-A-19534208 oder in Organikum, VEB Deutscher Verlag der Wissenschaften, Berlin 1988, 17. Aufl., S. 419 oder in Jerry March, Advanced Organic Chemistry, 3. Aufl., John Wiley and Sons, S. 338 ff. beschrieben . Vorzugsweise erfolgt die Hydrolyse zum Amin durch Umsetzung mit einer Base. Geeignete Basen sind beispielsweise Alkalihydroxide, wie Natriumhydroxid und Kaliumhydroxid, Erdalkalihydroxide, wie Calciumhydroxid, Alkali- und Erdalkalicarbonate, wie Natrium-, Kalium und Calciumcarbonat, Ammoniak, Amine, wie Dimethylamin, Trimethylamin, Diethylamin, Triethylamin, Diisopropylamin und Diisopropylethylamin, oder Aminoalkohole, wie Ethanolamin, Diethanolamin und Triethanolamin. Besonders bevorzugt verwendet man die genannten Alkalihydroxide, gegebenenfalls in Kombination mit einem Amin oder Aminoalkohol.

Die Hydrolyse kann in Wasser oder in einem organischen Lösungsmittel oder in einer Mischung aus Wasser und organischem Lösungsmittel durchgeführt werden. Geeignete organische Lösungsmittel sind Alkohole, vorzugsweise mit 1 bis 3 Kohlenstoffatomen, wie Methanol, Ethanol, Propanol oder Isopropanol, Glykole, insbesondere mit 2 bis 8 Kohlenstoffatomen, wie Ethylenglykol, Di- und Triethylenglykol, Amine und Aminoalkohole, z.B. die zuvor genannten Amine und Aminoalkohole, außerdem die Gemische der vorstehend genannten Lösungsmittel sowie deren Gemische mit Wasser. Die Hydrolyse erfolgt vorzugsweise bei erhöhter Temperatur, z.B. bei der Siedetemperatur des verwendeten Lösungsmittels.

Das Reaktionsprodukt kann durch übliche Verfahren aufgereinigt werden, beispielsweise durch Destillation, Sublimierung, Extraktion oder Chromatographie.

Liegt das erhaltene hydrolysierte Enantiomer der Verbindung (I) in Form des Ammoniumsalzes vor, beispielsweise weil das acylierte Produkt mit Säuren hydrolysiert wurde, wird das Amin aus dem Ammoniumsalz mittels einer geeigneten Base freigesetzt, wie oben für das im Wesentlichen nicht acylierte Enantiomer beschrieben. Auch hier kann das gewonnene freie Amin anschließend gegebenenfalls weiteren dem Fachmann bekannten Reinigungsschritten unterworfen werden.

Analog lassen sich bei Einsatz des cis-Steroisomers auch die (R,S)- und (S,R)-Enantiomere der Verbindung (I) herstellen.

In dem erfindungsgemäßen Verfahrens werden die in Schritt (b) oder (c) erhaltenen optisch aktiven Verbindungen der Formeln ((R,R)-I) oder ((S,S)-I) zur Erhöhung der chemischen und/oder optischen Reinheit gewünschtenfalls in ihre Ammoniumsalze durch Zugabe eines sauren Salzbildners überführt (Schritt (d)), als solche isoliert (Schritt (e)) und anschließend als freie Base wieder freigesetzt (Schritt (f)). Diese Schritte bieten sich insbesondere dann an, wenn die optische oder chemische Reinheit der in den Schritten (b) und (c) erhaltenen Enantiomere noch nicht zufriedenstellend ist.

Die Salzbildung erfolgt vorteilhafterweise unter Präzipitation des Säureadditionssalzes der Verbindungen ((R,R-I) oder ((S,S)-I). Betreffend die Durchführung und insbesondere die Auswahl des sauren Salzbildners gelten die oben zur Trennung durch Fällung des im Wesentlichen nicht acylierten Enantiomers (Schritt (b)) gemachten Angaben. Vorzugsweise legt man dazu das zu reinigende Enantiomer in einem organischen Lösungsmittel vor und versetzt die Lösung mit dem sauren Salzbildner. Man kann natürlich auch den sauren Salzbildner, gegebenenfalls im Lösungsmittel, vorlegen und mit dem Enantiomer versetzen, die erste Variante ist jedoch bevorzugt. Das Lösungsmittel wird vorzugsweise so gewählt, dass die freie Base, d.h. die Aminform des zu reinigenden Enantiomers (R,R)-I) oder ((S,S)-I), darin löslich ist, deren Ammoniumsalz hingegen im Wesentlichen nicht, d.h. zu höchstens 5 %, bevorzugt zu höchstens 2 % und insbesondere zu höchstens 1 %. Geeignete Lösungsmittel sind beispielsweise aliphatische und alicyclische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit 1 oder 2 Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Dioxan, oder Gemische der vorgenannten Lösungsmittel. Besonders bevorzugt werden die vorgenannten Ether und aromatischen Kohlenwasserstoffe eingesetzt. Speziell verwendet man Toluol.

Bei der Fällung der Ammoniumsalze der Verbindung der Formel (I) ist die Zugabe des sauren Salzbildners in Form einer wässrigen Lösung besonders bevorzugt. Bevorzugt verwendet man wässrige Lösungen von Mineralsäuren, wie Salzsäure, Bromwasserstoffsäure, Schwefelsäure, Salpetersäure oder Phosphorsäure. Insbesondere eignet sich Salzsäure als saurer Salzbildner. Dies ist überraschend, da trotz der Anwesenheit von Wasser eine selektive Präzipitation des Ammoniumsalzes des zu reinigenden Enantiomers ohne wesentliche Ausbeuteverluste stattfindet.

In dieser speziellen Ausführungsform des erfindungsgemäßen Verfahrens kann es von Vorteil sein, das mit dem sauren Salzbildner zugegebene Wasser durch eine dem Fachmann bekannte Methode vollständig oder wenigstens teilweise wieder zu entfernen. Beispielsweise eignet sich hierzu in Abhängigkeit des Lösungsmittels die azeotrope Destillation.

Vorteilhafterweise lassen sich Reinheit und Ausbeute des aus der Fällung erhaltenen Salzes der Verbindung der Formel (I), in Abhängigkeit der zu erfüllenden Spezifikationen des Endproduktes, über den Wassergehalt des Reaktionsgemisches gezielt beeinflussen.

Die Isolierung der gebildeten Ammoniumsalze erfolgt durch allgemein übliche Verfahren zur Abtrennung von präzipitierten Feststoffen, z.B. durch Filtration, Sedimentieren, Zentrifugieren etc. mit oder ohne vorherige Entfernung der im Reaktionsgemisch vorhandenen flüssigen Phase. Letztere kann z.B. durch Dekantieren oder Destillation entfernt werden. Das abgetrennte Ammoniumsalz kann gewünschtenfalls weiteren Reinigungsschritten unterworfen werden, wie Waschen oder Digerieren mit einem Lösungsmittel, in welchem das Salz nicht löslich ist.

Die im Wesentlichen enantiomerenreinen Verbindungen (I) lassen sich nach Isolierung des Fällungsprodukts aus ihren Ammoniumsalzen durch Neutralisation mit einer Base wieder freisetzen. Die zu Schritt (b) gemachten Angaben zu geeigneten Basen gelten hier entsprechend. Insbesondere erfolgt die Neutralisation mit einer wässrigen NaOH-Lösung.

Durch diese Ausführungsform lässt sich die optische und chemische Reinheit der im Wesentlichen enantiomerenreinen Verbindungen (I) (insbesondere (R,R)-I und (S,S)-I) weiter erhöhen. Auch die chemische und/oder optische Reinheit von optisch aktiven Verbindungen der Formel (I), die aufgrund ihrer zu geringen Reinheit im Rahmen der vorliegenden Erfindung nicht als im Wesentlichen enantiomerenreine Verbindungen (I) bezeichnet werden, lässt sich auf diese Weise erhöhen.

Durch diese Ausführungsform lassen sich chemische bzw. optische Reinheiten der 2-Benzyloxycyclohexylaminenantiomere von wenigstens 99 % bzw. wenigstens 99 % ee, bevorzugt wenigstens 99,5 % bzw. wenigstens 99,5 % ee und insbesondere wenigstens 99,9 % bzw. wenigstens 99,9 % ee erreichen.

Die erreichbaren Ausbeuten bei der Fällung der Verbindungen (I) in Form ihrer Ammoniumsalze durch Zugabe eines sauren Salzbildners zur Erhöhung der chemischen und/oder optischen Reinheit sind abhängig von der chemischen und optischen Reinheit der Ausgangsverbindungen sowie von den angestrebten Reinheiten des hieraus erhaltenen Produkts. Der Ausbeuteverlust liegt bevorzugt bei weniger als 15 % und insbesondere bei weniger als 10 %, bezogen auf die Gesamtmenge des zu isolierenden Enantiomers.

In einer weiteren bevorzugten Ausführungsform des erfindungsgemäßen Verfahrens wird das trans-Stereoisomer der Verbindung der Formel (I) dadurch erhalten, dass man:
(i) Cyclohexenoxid (Epoxycyclohexan) mit Ammoniak unter Erhalt der Verbindung der Formel (II) 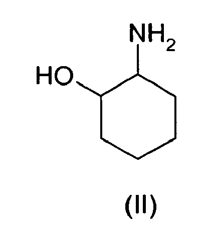 umsetzt; und
(ii) das trans-Stereoisomer der Verbindung der Formel (II) mit einer geeigneten Benzylverbindung zum trans-Stereoisomer der Verbindung der Formel (I) umsetzt oder
(iii) ein Gemisch aus cis- und trans-Isomer der Verbindung der Formel (II) mit einer geeigneten Benzylverbindung zu einem Gemisch aus den entsprechenden cis- und trans-Benzylethern umsetzt (d.h. zu einem Gemisch der trans- und der cis-Isomere der Verbindung I) und das trans-Stereoisomer der Verbindung der Formel (I) daraus isoliert.

Die im Rahmen der vorliegenden Erfindung als Schritt (i) bezeichnete Herstellung von Aminocyclohexanol aus Cyclohexenoxid kann beispielsweise gemäß der von Schlichter und Frahm veröffentlichten Vorschrift (Arch. Pharm. (Weinheim) 326, 429-436 (1993)) durchgeführt werden. Darin wird Cyclohexenoxid zu einer wässrigen 35 - 40%igen Ammoniaklösung gegeben, über Nacht gerührt und die Lösung zur Trockne eingeengt. Die Reaktion verläuft dabei in der Regel stereospezifisch und führt selektiv zu trans-2-Aminocyclohexanol.

Die Überführung der Verbindung (II) in den entsprechenden Benzylether erfolgt in Schritt (ii) durch Umsetzung des trans-Isomers der Verbindung (II) mit einer geeigneten Benzylverbindung. Geeignete Benzylverbindungen sind beispielsweise Benzylhalogenide, z.B. Benzylbromid oder Benzylchlorid. Die Umsetzung wird in der Regel in Gegenwart einer Base durchgeführt. Geeignete Basen sind die zuvor genannten Alkali- und Erdalkalihydroxide sowie Alkali- und Erdalkalicarbonate. Das so erhaltene Enantiomerengemisch kann gegebenenfalls durch dem Fachmann bekannte Methoden isoliert und aufgereinigt werden. Hierfür eignen sich insbesondere destillative und extraktive Methoden.

In der Regel wird durch die oben beschriebene Reaktionsfolge in Schritt (i) im Wesentlichen das trans-Stereoisomer der Verbindung (II) und im nachfolgenden Schritt (ii) im Wesentlichen das trans-Stereoisomer der Verbindung (I) erhalten, insbesondere mit einem Diastereomerenüberschuss von wenigstens 99 % de. Speziell handelt es sich bei dem isolierten Enantiomerengemisch um das Racemat des trans-Diastereomers der Verbindung (I). Sollte das Reaktionsgemisch in Schritt (i) jedoch eine nicht unerheblichen Menge an cis-Isomer, d.h. mehr als 1 %, bezogen auf das Gesamtgewicht der cis/trans-Isomere, enthalten, wird dieses vorzugsweise mittels geeigneter Verfahren vom trans-Isomer abgetrennt. Dem Trennverfahren wird in der Regel das Reaktionsgemisch des Schritts (i) unterworfen; d.h. vor der Durchführung des Schrittes (ii) wird das Reaktionsgemisch des Schrittes (i) vom cis-Isomeren befreit: Alternativ ist es jedoch auch möglich, das in Schritt (i) erhaltene Gemisch erst Schritt (iii) zu unterwerfen, d.h. das cis/trans-Gemisch erst mit einer geeigneten Benzylverbindung umzusetzen, und dann das Reaktionsgemisch dieser Benzylierungsreaktion aufzutrennen. Das Reaktionsgemisch der Benzylierungsreaktion umfasst die trans- und cis-Isomere der Verbindung I; aus diesem Gemisch wird das trans-Isomer isoliert. Bezüglich geeigneter und bevorzugter Benzylverbindungen und Reaktionsbedingungen für die Benzylierung wird auf die obigen Ausführungen verwiesen. Geeignete Trennverfahren sind dem Fachmann bekannt und umfassen beispielsweise extraktive und chromatographische Methoden.

Das so erhaltene trans-Stereoisomer der Verbindung (I) kann isoliert und gegebenenfalls weiter aufgereinigt werden, beispielsweise chromatographisch, extraktiv oder durch Fällung als Ammoniumsalz.

Mit dem erfindungsgemäßen Verfahren sind die einzelnen Enantiomere der Verbindung (I) mit einer sehr hohen optischen und chemischen Reinheit und in hohen Ausbeuten erhältlich. Speziell wird mit dem erfindungsgemäßen Verfahren (R,R)-2-Benzyloxycyclohexylamin mit einem Enantiomerenüberschuss (ee-Wert) von vorzugsweise wenigstens 98% ee, besonders bevorzugt von wenigstens 99% ee, stärker bevorzugt wenigstens 99,5% ee und insbesondere von wenigstens 99,9% ee erhalten. Die Enantiomerenreinheit des entsprechenden (S,S)-Enantiomers beträgt vorzugsweise wenigstens 97% ee, besonders bevorzugt wenigstens 98% ee, stärker bevorzugt wenigstens 99% ee, noch stärker bevorzugt wenigstens 99,5% ee und insbesondere wenigstens 99,9% ee. Die chemische Reinheit der Enantiomere beträgt vorzugsweise wenigstens 97%, besonders bevorzugt wenigstens 98%, stärker bevorzugt wenigstens 99%, noch stärker bevorzugt wenigstens 99,5% und insbesondere wenigstens 99,9%. Der Enantiomerenüberschuss der Amine ((R,R)-I) und ((S,S)-I) kann mittels gängiger Verfahren bestimmt werden, beispielsweise durch Bestimmung des optischen Drehwerts oder durch Chromatographie an einer chiralen Phase, beispielsweise durch HPLC oder Gaschromatographie über chirale Säulen.

Die vorliegende Erfindung wird durch die nachfolgenden Beispiele veranschaulicht.

### Beispiele

### 1.) Enantioselektive Acylierung und Racematspaltung

Racemisches trans-2-Benzyloxycyclohexylamin (1537 g, 7,49 mol), bestehend zu gleichen Teilen aus (R,R)-2-Benzyloxycyclohexylamin und (S,S)-2-Benzyloxycyclohexylamin, wurde gemeinsam mit Methoxyessigsäureisopropylester (MEI-PE) (425 g, 3,97 mol, 0,53 Äquivalente) vorgelegt, auf 15° C gekühlt und anschließend mit Novozyme 435® (30 g) versetzt. Nach Entfernen des Kühlbads wurde die Reaktionslösung über Nacht gerührt, wobei die Rührgeschwindigkeit so eingestellt wurde, dass das Enzym gerade in der Schwebe blieb. Nach 20 Stunden wurde das Enzym abfiltriert, der Filterrückstand mit Toluol (0,5 I) gewaschen und das Filtrat am Vakuum (15 mbar) bei einer maximalen Temperatur von 50°C von flüchtigen Bestandteilen befreit. Der Destillationsrückstand wurde in Toluol (2,5 I) aufgenommen und unter Kühlung durch Zugabe von 10%iger Schwefelsäure (ca. 1,8 I) auf einen pH-Wert von 1 gebracht, wobei darauf geachtet wurde, dass die Temperatur 20°C nicht überschritt. Die wässrige Phase wurde abgetrennt und zweimal mit Toluol (je 200 ml) extrahiert. Die vereinten organischen Extrakte wurden mit 10%iger Schwefelsäure (100 ml) und Wasser (200 ml) gewaschen. Das Lösungsmittel wurde daraufhin unter vermindertem Druck entfernt und der Destillationsrückstand bei 0,5 mbar und einer Badtemperatur von 180°C von niedersiedenden Bestandteilen befreit. Als Rückstand wurde (R,R)-Methoxyessigsäure-2-benzyloxycyclohexylamid (920 g, 89 %) als hochviskoses Öl erhalten. Umkristallisieren aus heißem Cyclohexan und n-Pentan lieferte das Produkt als kristallinen Feststoff (Schmelzpunkt 45 - 48°C). Der ee-Wert betrug >99,9%.Das (S,S)-Enantiomer befand sich im Wesentlichen in Form seines Salzes in den vereinten wässrigen Phasen der Extraktion.
¹H-NMR (400 MHz, CDCl₃) von (R,R)-Methoxyessigsäure-2-benzyloxycyclohexyl-amid : δ = 1,22 (m, 2H), 1,38 (m, 2H), 1,58 - 1,68 (m, 1 H), 1,80 (m, 2H), 2,15 (m, 2H), 3,25 (m, 1 H), 3,40 (s, 3H), 3,84 und 3,92 (J_{AB} = 20 Hz, 2H), 3,05 (m, 1 H), 4,48 und 4,68 (J_{AB} = 16Hz, 2H), 6,55 (s, breit, 1 H), 7,22 - 7,40 (m, 5H).

### 2.) Isolierung der Enantiomere

### 2.1) Isolierung von (S,S)-2-Benzyloxycyclohexylamin

Die unter 1 erhaltenen, vereinten wässrigen Phasen wurden mit Toluol (2 I) versetzt. Der pH-Wert wurde mit wässriger NaOH-Lösung (50%ig) auf etwa 13 eingestellt, wobei das Gemisch durch Kühlung bei einer Temperatur von weniger als 30°C gehalten wurde. Nach Filtration über Glaswolle wurde die wässrige Phase abgetrennt und zweimal mit Toluol (je 200 ml) extrahiert. Die vereinten organischen Phasen wurden mit Wasser (200 ml) gewaschen. Anschließend wurde die wässrige Phase verworfen und das Lösungsmittel der organischen Phasen am Rotationsverdampfer entfernt. Als Rückstand wurde ein braunes Öl (950g), das neben Lösungsmittelresten als Hauptbestandteil 87 % (S,S)-2-Benzyloxycyclohexylamin enthielt, erhalten. Destillation unter vermindertem Druck (0,5 mbar/ 100°C) lieferte (S,S)-2-Benzyloxycyclohexylamin (653 g, 85%) mit einer chemischen Reinheit von 97% und einem ee-Wert von 97,3%.
¹H-NMR (400 MHz, CDCl₃): δ = 1,08 - 1,38 (m, 4H), 1,58 -1,82 (m, 4H), 1,90 (m, 1 H), 2,25 (m, 1 H), 2,65 (m, 1 H), 3,05 (m, 1H), 4,45 und 4,70 (J_{AB} = 16 Hz, 2H), 7,22-7,40 (m, 5H).

### 2.2) Herstellung von (R,R)-2-Benzyloxycyclohexylamin

(R,R)-Methoxyessigsäure-2-benzyloxycyclohexylamid (1687 g, 6,08 mol) wurde mit Triethanolamin (300 g) verdünnt und unter Rühren mit wässriger Natronlauge (50%ig, 780 g, 9,75 mol) versetzt. Das Gemisch wurde auf eine Innentemperatur von 120°C erhitzt und kräftig gerührt. Nach 7 Stunden wurde das Reaktionsgemisch mit Wasser (500 ml) verdünnt. Nach Abkühlen auf Raumtemperatur wurde Toluol (2 I) zugegeben und die Phasen voneinander getrennt. Die wässrige Phase wurde erneut mit Toluol (200 ml) extrahiert, die organischen Phasen wurden vereint und die vereinten organischen Phasen wurden mit Wasser (200 ml) gewaschen. Nach Abtrennen der wässrigen Phase wurde das Lösungsmittel am Rotationsverdampfer entfernt und der Rückstand unter vermindertem Druck fraktionierend destilliert (Siedepunkt 100°C, bei 0,4 bis 0,5 mbar). (R,R)-2-Benzyloxycyclohexylamin wurde als klare farblose Flüssigkeit in einer Ausbeute von 1125 g (90%) erhalten. Der ee-Wert betrug > 99,9 %.
Drehwert [α]_{D} = -96,8° (c = 2 in Methanol)
¹H-NMR-Spektrum identisch mit demjenigen des (S,S)-Enantiomers

### 3.) Erhöhung der chemischen und/oder optischen Reinheit

Diese folgenden Beispiele dienen zur Erläuterung der hohen Effizienz der erfindungsgemäßen Fällung.

### 3.1) Fällung von (S,S)-2-Benzyloxycyclohexylaminhydrochlorid

(S,S)-2-Benzyloxycyclohexylamin mit einer chemischen und optischen Reinheit von 98,5 % respektive 89,6% ee (eingestellt durch Zumischen des (R,R)-Enantiomers) wurde in Toluol (90 ml) vorgelegt und unter Rühren mit konzentrierter Salzsäure versetzt. Nach beendeter Zugabe wurde zum Rückfluss erhitzt und das Destillat über einen mit Toluol gefüllten Wasserabscheider in das Reaktionsgefäß zurückgeführt. Nach beendeter Wasserabscheidung wurde der Wasserabscheider gegen einen Rückflusskühler ausgetauscht und Isopropanol (10 ml) zugegeben. Nach Abkühlen auf Raumtemperatur wurde der ausgefallene Feststoff durch Filtration isoliert, mit kaltem Toluol (10 ml) gewaschen und unter vermindertem Druck getrocknet. Das Hydrochlorid von (S,S)-2-Benzyloxycyclohexylamin wurde als farbloser Feststoff (20,1 g, 83 %) mit einem Schmelzpunkt von 182 - 184 °C erhalten. Eine durch wässrige Natronlauge freigesetzte Probe des freien 2-Benzyloxycyclohexylamins besaß eine chemische und optische Reinheit von > 99,9% respektive > 99,9% ee.

### 3.2) Fällung von (S,S)-2-Benzyloxycyclohexylaminhydrochlorid

Bei Verwendung von (S,S)-2-Benzyloxycyclohexylamin mit einer optischen Reinheit von 85% ee (eingestellt durch Zumischen des (R,R)-Enantiomers) wurde unter Anwendung der Vorschrift aus Beispiel 3.1) mit einer Ausbeute von 82,5% ein Hydrochlorid erhalten, das eine optische Reinheit von 95% ee aufwies.

Die Wiederholung der Fällung führte zu einem Produkt mit >99%ee.

Drehwert des Ammoniumsalzes: [α]_{D} +78,8° (c = 2 in Methanol) ¹H-NMR (400 MHz, CDCl₃) des Hydrochlorids: δ = 1,05 - 1,38 (m = 3H), 1,70 (m, 3H), 2,10 (m, 1 H), 2,35 (m, 1 H), 3,05 (m, 1 H), 3,55 (m, 1 H) 4,50 und 4,70 (J_{AB} = 16 Hz, 2H), 7,25 - 7,50 (m, 5H), 8,50 (s, breit, 3H).

### 3.3) Fällung von (R,R)-2-Benzyloxycyclohexylaminhydrochlorid

Ausgehend von (R,R)-2-Benzyloxycyclohexylamin mit einer chemischen Reinheit von 96% und einer optischen Reinheit von > 99,9%ee wurde das Hydrochlorid (324,3 g, 85%) als farbloser Feststoff erhalten. Eine durch wässrige Natronlauge freigesetzte Probe des freien Amins besaß eine chemische und optische Reinheit von > 99,9% respektive > 99,9% ee.

## Patentansprüche

1. Verfahren zur Herstellung von optisch aktiven Verbindungen der Formeln ((R,R)-I) und/oder ((S,S)-I) 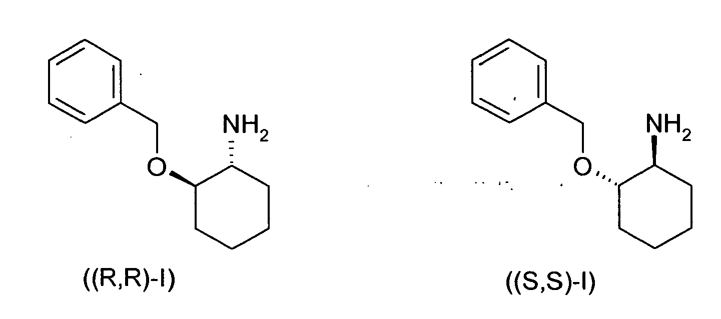 umfassend die folgenden Schritte:
(a) Umsetzung eines Enantiomerengemischs des trans-Stereoisomers der Verbindung der Formel (I) 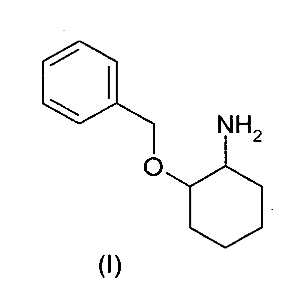 mit einem Acylierungsmittel in Gegenwart einer Hydrolase, wobei man ein Gemisch erhält, in welchem ein Enantiomer des trans-Stereoisomers der Verbindung (I) im Wesentlichen in der acylierten Form vorliegt und das andere Enantiomer des trans-Stereoisomers der Verbindung (I) im Wesentlichen in der nicht acylierten Form vorliegt;
(b) Abtrennung des nicht acylierten Enantiomers des trans-Stereoisomers der Verbindung (I) aus dem in Schritt (a) erhaltenen Gemisch;
(c) Hydrolyse des in Schritt (b) erhaltenen im Wesentlichen acylierten Enantiomers des trans-Stereoisomers der Verbindung (I) zum entsprechenden nicht acylierten Enantiomer des Amins (I);
(d) Überführung der in Schritt (b) oder (c) erhaltenen optisch aktiven Verbindungen der Formeln ((R,R)-I) oder ((S,S)-I) durch Zugabe eines sauren Salzbildners in ihre Ammoniumsalze, wobei der saure Salzbildner in Form einer wässrigen Lösung zugegeben wird und wobei nach Zugabe der wässrigen Lösung das Wasser wenigstens teilweise aus der Reaktionslösung entfernt wird;
(e) Isolierung der Ammoniumsalze; und
(f) Freisetzung der freien Basen der Formeln ((R,R)-I) oder ((S,S)-I) aus den Ammoniumsalzen.

2. Verfahren nach Anspruch 1, wobei es sich bei dem in Schritt (a) erhaltenen im Wesentlichen nicht acylierten Enantiomer um das (S,S)-Enantiomer der Verbindung (I) handelt und es sich bei dem im Wesentlichen acylierten Enantiomer um das (R,R)-Enantiomer der Verbindung (I) handelt.

3. Verfahren nach einem der vorhergehenden Ansprüche, wobei das Acylierungsmittel ausgewählt ist unter Estern, deren Säurekomponente in α-, β- oder γ-Stellung zum Carbonyl-Kohlenstoffatom eine Sauerstoff-, Stickstoff-, Fluor- oder Schwefel-haltige Gruppe trägt.

4. Verfahren nach einem der vorhergehenden Ansprüche, wobei das Acylierungsmittel in Schritt (a) in einer Menge von 1,0 bis 1,5 Äquivalenten bezogen auf die molare Menge des zu acylierenden Enantiomers eingesetzt wird.

5. Verfahren nach einem der vorhergehenden Ansprüche, wobei die Hydrolase ausgewählt ist unter Lipasen aus Bakterien der Gattung Burkholderia oder Pseudomonas oder aus Hefen der Gattung Candida.

6. Verfahren nach Anspruch 5, wobei es sich bei der Lipase um Lipase B aus Candida antarctica handelt.

7. Verfahren nach einem der vorhergehenden Ansprüche, wobei man die Umsetzung mit dem Acylierungsmittel in Gegenwart der Hydrolase ohne Zusatz von Lösungsmitteln durchführt.

8. Verfahren nach einem der Ansprüche 1 bis 6, wobei man die Umsetzung mit dem Acylierungsmittel in Gegenwart der Hydrolase in einem nichtwässrigen Reaktionsmedium durchführt.

9. Verfahren nach einem der vorhergehenden Ansprüche, wobei der saure Salzbildner ausgewählt ist unter Protonensäuren und deren Alkalimetallsalzen.

10. Verfahren nach einem der vorhergehenden Ansprüche, wobei das trans-Stereoisomer der Verbindung der Formel (I) **dadurch** erhalten wird, dass man:
(i) Cyclohexenoxid mit Ammoniak unter Erhalt der Verbindung der Formel (II) 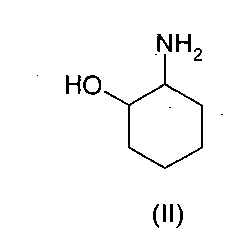 umsetzt; und
(ii) das trans-Stereoisomer der Verbindung der Formel (II) mit einer geeigneten Benzylverbindung zum trans-Stereoisomer der Verbindung der Formel (I) gemäß Anspruch 1 umsetzt oder
(iii) ein Gemisch aus cis- und trans-Isomer der Verbindung der Formel (II) mit einer geeigneten Benzylverbindung zu einem Gemisch aus den entsprechenden cis- und trans-Benzylethem umsetzt und das trans-Stereoisomer daraus isoliert.

## Claims

1. A process for preparing optically active compounds of the formulae ((R,R)-I) and/or ((S,S)-I) 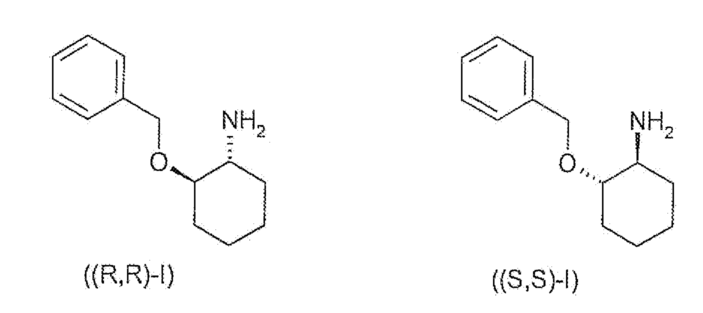 comprising the following steps:
(a) reacting a mixture of enantiomers of the trans stereoisomer of the compound of the formula (I) 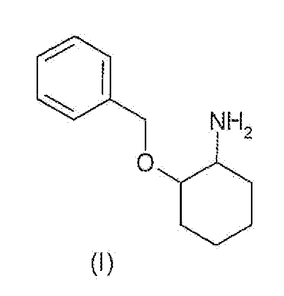 with an acylating agent in the presence of a hydrolase, resulting in a mixture in which one enantiomer of the trans stereoisomer of the compound (I) is present substantially in the acylated form, and the other enantiomer of the trans stereoisomer of the compound (I) is present substantially in the
nonacylated form;
(b) removing the nonacylated enantiomers of the trans stereoisomer of the compound (I) from the mixture obtained in step (a);
(c) hydrolyzing the substantially acylated enantiomer of the trans stereoisomer of the compound (I), obtained in step (b), to the corresponding nonacylated enantiomers of the amine (I);
(d) converting the optically active compounds of the formulae ((R,R)-I) or ((S,S)-I) obtained in step (b) or (c) into their ammonium salts by adding an acidic salt former, where the acidic salt former is added in the form of an aqueous solution where the water is removed at least in part from the reaction solution after addition of the aqueous solution;
(e) isolating the ammonium salts; and
(f) liberating the free bases of the formulae ((R,R)-I) or ((S,S)-I) from the ammonium salts.

2. The process according to claim 1, where the substantially, nonacylated enantiomer obtained in step (a) is the (S,S) enantiomer of the compound (I), and the substantially acylated enantiomer is the (R,R) enantiomer of the compound (I).

3. The process according to any of the preceding claim, where the acylating agent is selected from esters whose acid component has an oxygen, nitrogen-, fluorine- or sulfur-containing group in the α,β or γ position relative to the carbonyl carbon atom.

4. The process according to any of the preceding claims, where the acylating agent is employed in step (a) in an amount of from 1.0 to 1.5 equivalents based on the molar amount of the enantiomer to be acylated.

5. The process according to any of the preceding claims, where the Hydrolase is selected from lipases from bacteria of the genus Burkholderia or Pseudomonas or from yeasts of the genus Candida.

6. The process according to claim 5, where the lipase is Lipase B from Candida antarctica.

7. The process according to any of the preceding claims, where the reaction with the acylating agent is carried out in the presence of the hydrolase without addition of solvents.

8. The process according to any of claims 1 to 6, where the reaction with the acylating agent is carried out in the presence of the hydrolase in a nonaqueous reaction medium.

9. The process according to any of the preceding claims, where the acidic salt former is selected from protic acids and their alkali mental salts.

10. The process according to any or the preceding claim, where the trans stereoisomer of the compound of the formula (I) is obtained by:
(i) reacting cyclohaxana oxide with ammonia to result in the compound of the formula (II) 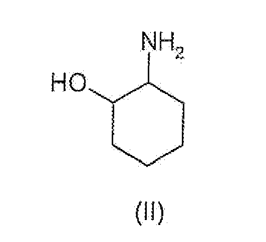 and
(ii) reacting the trans stereoisomer of the compound of the formula (II) with a suitable benzyl compound to give the trans stereoisomer of the compound of the formula (I) according to claim or
(iii) reacting a mixture of cis- and transisomers of the compound of the formula (II) with a suitable benzyl compound to form a mixture of the corresponding cis- and trans-benzyl ethers and isolating therefrom the trans-stereoisomer.

## Revendications

1. Procédé de fabrication de composés optiquement actifs de formules ((R,R)-I) et/ou ((S,S)-I) 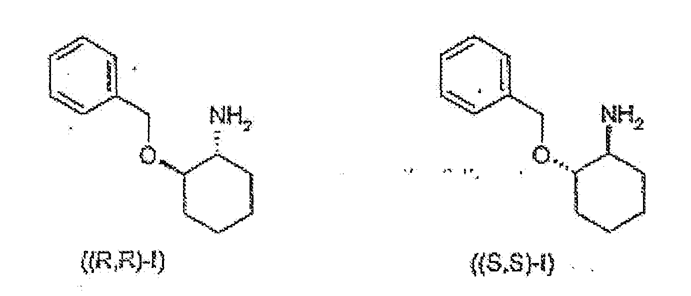 comprenant les étapes suivantes :
(a) la mise en réaction d'un mélange d'énantiomères du stéréoisomère trans du composé de formule (I) 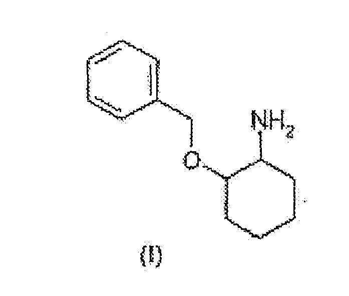 avec un agent d'acylation en présence d'une hydrolase, un mélange étant obtenu, dans lequel un énantiomère du stéréoisomère trans du composé (I) se présente essentiellement sous la forme acylée et l'autre énantiomère du stéréoisomère trans du composé (I) se présente essentiellement sous la forme non acylée ;
(b) la séparation de l'énantiomère non acyle du stéréoisomère trans du composé (I) du mélange obtenu à l'étape (a) ;
(c) l'hydrolyse de l'énantiomère essentiellement acylé du stéréoisomère trans du composé (I) obtenu à l'étape (b) en l'énantiomère non acyle correspondant de l'amine (I) ;
(d) la conversion des composés optiquement actifs de formules ((R,R)-I) ou ((S,S)-I) obtenus à l'étape (b) ou (c) en leurs sels d'ammonium par ajout d'un agent de formation de sel acide, l'agent de formation de sel acide étant ajouté sous la forme d'une solution aqueuse et l'eau étant éliminée au moins en partie de la solution réactionnelle après l'ajout de la solution aqueuse ;
(e) l'isolement des sels d'ammonium ; et
(f) la libération des bases libres de formules ((R,R)-I) ou ((SS)-I) à partir des sels d'ammonium.

2. Procédé selon la revendication 1, dans lequel l'énantiomère essentiellement non acylé obtenu à l'étape (a) est l'énantiomère (S,S) du composé (I) et l'énantiomère essentiellement acylé est l'énantiomère (R,R) du composé (I).

3. Procédé selon l'une quelconque des revendications précédentes, dans lequel l'agent d'acylation est choisi parmi les esters dont le composant acide porte en position α, β ou γ par rapport à l'atome de carbone du carbonyle un groupe contenant de l'oxygène, de l'azote, du fluor ou du soufre.

4. Procédé selon l'une quelconque des revendications précédentes, dans lequel l'agent d'acylation est utilisé à l'étape (a) en une quantité de 1,0 à 1,5 équivalents par rapport à la quantité molaire de l'énantiomère à acyle.

5. Procédé selon l'une quelconque des revendications précédentes, dans lequel l'hydrolase est choisie parmi les lipases de bactéries du genre Burkholderia ou Pseudomonas ou les levures du genre Candida.

6. Procédé selon la revendication 5, dans lequel la lipase est la lipase B de Candida antarctica.

7. Procédé selon l'une quelconque des revendications précédentes, dans lequel la réaction avec l'agent d'acylation en présence de l'hydrolase est réalisée sans ajout de solvants.

8. Procédé selon l'une quelconque des revendications 1 à 6, dans lequel la réaction avec l'agent d'acylation en présence de l'hydrolase est réalisée dans un milieu de réaction non aqueux.

9. Procédé selon l'une quelconque des revendications précédentes, dans lequel l'agent de formation de sel acide est choisi parmi les acides protoniques et leurs sels de métaux alcalins.

10. Procédé selon l'une quelconque des revendications précédentes, dans lequel le stéréoisomère trans du composé de formule (I) est obtenu par :
(i) la mise en réaction d'oxyde de cyclohexène avec de l'ammoniac pour obtenir le composé de formule (II) 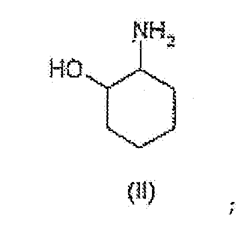 et
(ii) la mise en réaction du stéréoisomère trans du composé de formule (II) avec un composé de benzyle approprié pour former le stéréoisomère trans du composé de formule (I) selon la revendication 1 ou
(iii) la mise en reaction d'un mélangé de l'isomère cis et de l'isomère trans du composé de formule (II) avec un composé de benzyl approprié pour former un mélange des éthers de benzyle cis et trans correspondants et l'isolement du stéréoisomère trans à partir de celui-ci.
